# EUROPEAN PATENT APPLICATION

(11) **EP 2 390 346 A1**
(43) Date of publication of application: **30.11.2011**
(21) Application number: 10164387.2
(22) Date of filing: 28.05.2010
(51) Int. Cl.: C12Q 1/68, C12N 5/07

(54) **Osteoarthrosis markers**

(71) Applicant: UNIVERSITEIT TWENTE, 7522 NB Enschede (NL)
(72) Inventor: Karperien, Hermanus Bernardus Johannes, 7151 DJ Eibergen (NL); Leijten, Jeroen Christianus Hermanus, 7523 RG Enschede (NL)
(74) Representative: Hatzmann, Martin

(57) **Abstract**

The invention relates to a method for determining whether an individual has an increased risk of developing symptoms of a disease affecting a joint or of developing symptoms associated with progression of said a disease affecting a joint, within a time frame of 10 years after the completion of said method compared to the average risk of a reference population, comprising determining in a sample of said individual the levels of gene expression products of Grem1, FRZb and DKK1 and determining whether the gene expression products are present at a level that is different when compared to the level of the same gene expression products of in a sample of a control.

## Description

### TECHNICAL FIELD

The present invention relates to identification of genes related to joint diseases such as osteoarthritis, diagnosis of genetic susceptibility to the diseases, and the like.

### BACKGROUND OF THE INVENTION

Lifestyle-related diseases of bone and joints have direct effects on life in only a few cases, they represent the major cause of deterioration of the quality of life of the elderly because they interfere with the activities of daily living due to pain, gait disturbance and the like. Also, because these diseases are characterized by rapid rises in incidence with aging and by a chronic course, they impose a great burden on national medical economics, posing an important problem to be overcome in aging society as a whole. The World Health Organization (WHO) positioned the first 10 years of the 21 century as "The Bone and Joint Decade" and begun efforts to conquer bone and joint diseases.

Osteoarthritis, a bone and joint disease accompanied by chronic arthritis, is characterized by cartilage destruction and progressive changes in bone and cartilage due to regressive degeneration of cartilage. It is reported that not less than 80% of the people aged 60 years or more exhibit symptoms of osteoarthritis in their knees, elbows, hip joints, and spine, and this disease is a representative common disease. At present, no fundamental therapeutic approach for Osteoarthritis is available, with symptomatic therapies for pain relief, such as administration of non-steroidal anti-inflammatory analgesics, hyaluronic acid, or steroid agents, forming the mainstream. For advanced cases, surgeries such as arthroscopic surgery, osteotomy, or prosthetic replacement are indicated; however, because of the limited durability of joint prosthesis, it is thought to be desirable to avoid this surgery until about 55 years of age.

Osteoarthritis is a multifactor disease influenced by environmental factors and genetic factors. It is of great importance that diseases of the joints and in particular Osteoarthritis are detected at an early stage and preferably even before symptoms occur. A lot of effort has been put into developing biochemical markers that can aid early-stage joint diseases, predicting such diseases and assessing therapeutic response.

A number of biochemical biomarkers have been identified which can be used as indicators of pathogenic processes of a joint. However, no breakthrough has been achieved and practical use of existing biochemical markers has been limited due to their poor predictive value. Biochemical markers for osteoarthritis have been reviewed for example in W.E. van Spil et al, Osteoarthritis and Cartilage xxx (2010) 1-8, and in J-C Rousseau and P. D. Delmas, Nature Clin Pract. (2007) Vol. 3 No. 6, 346-356.

Therefore, there is a demand for biochemical biomarkers which are indicative of diseases of the joints.

### SUMMARY OF THE INVENTION

The invention is based on the surprising finding that levels of gene expression products of the genes Grem1, FRZb and DKK1 in a sample originating from a joint are strongly correlated with the condition of the joint. The invention therefore provides a method for evaluating the condition of a sample obtained from an individual, which sample comprises gene expression products of a cell residing in a joint of said individual, preferably a chondrocyte, said method comprising determining in said sample the expression levels of the genes Grem1, FRZb and DKK1. Preferably, said determining is done simultaneously in a multiplex assay, because multiplex assays are more reliable and accurate than the combined results of the measurements of the individual levels of Grem1, FRZb and DKK1 and are more suitable for high throughput applications.

Preferably, said method further comprises determining whether the said expression levels differ in comparison to the corresponding expression levels in a sample of a healthy control individual or in a sample of an unaffected joint (preferably the lateral joint) of the same individual.

In another embodiment, the invention provides a method which further comprising determining in said sample the gene expression level of at least one of the genes selected from the group of LTBP1, LTBP2, ID2, ID3, GDF10, Smad3, TGFβ3, Wnt5A, Wnt10B, Wnt11, FZD6, FZD8, FZD10, LEF1, GSK3B, Co13A1, Co15A2, KAL1, Co16A3, FBN1, Co114A1 Matn1, Co112A1, Co14A5, MFAP3, FBN3, Co14A6, CPXM1, CILP, DLK1, SRPX2, CHRDL2, LTBP2, CFH, MMP2, Wnt5A and WFDC1, genes coding for a Bone Morphogenic Protein (BMP) antagonist and genes coding for a Wnt antagonist. Preferably, it is determined whether said gene expression level is present at a level that is different when compared to the gene expression level of the same genes in a sample of a healthy control individual or in a sample of an unaffected joint (preferably the lateral) of the same individual. In case said gene expression products of said at least one of the genes selected from the above mentioned group are proteins, said group comprises CPXM1, CILP, DLK1, SRPX2, CHRDL2, LTBP2, CFH, MMP2, Wnt5A enWFDC1 and genes coding for a Bone Morphogenic Protein (BMP) antagonist and genes coding for a Wnt antagonist, because the proteins encoded by these genes are secreted. An advantage thereof is that the method according to the invention is even more accurate. Another advantage is that determining the expression levels of secreted products can be done in easily accessible samples. Preferably, said sample is a saliva, a blood, a serum, a urine or a synovial fluid sample. In some preferred embodiments, said sample comprises a cell originating from articular cartilage, preferably a chondrocyte.

Preferably, said individual is a mammal, preferably a human.

In some preferred embodiments, a method according to the invention further comprises a step of determining the levels of the uCTX-II and/or sCOMP proteins.

The invention further provides a method for determining whether an individual has an increased risk of having a joint disease, or developing symptoms of a joint disease or developing symptoms associated with progression of a joint disease, wherein said symptoms develop within 10 years after the completion of said method, wherein said method comprises performing the method of any one of claims 1-7 and further determining whether said condition is associated with an increased risk compared to the average risk of a reference population based on the comparison of the gene expression levels.

Preferably, said joint disease comprises osteoarthritis, arthritis or rheumatoid arthritis (RA).

In another aspect, the invention relates to a kit of parts comprising up to 50 reagents, sequence specific oligonucleotides and/or capture agent for detecting Grem1, FRZb and DKK1. More preferably, said kit further comprises at least one of the gene expression products of the genes LTBP1, LTBP2, ID2, ID3, GDF10, Smad3, TGFβ3, Wnt5A, Wnt10B, Wnt11, FZD6, FZD8, FZD10, LEF1, GSK3B, Col3A1, Co15A2, KAL1, Co16A3, FBN1, Co114A1 Matn1, Co112A1, Co14A5, MFAP3, FBN3, Co14A6, CPXM1, CILP, DLK1, SRPX2, CHRDL2, LTBP2, CFH, MMP2, Wnt5A and WFDC1. Preferably said gene expression products are proteins. Preferably, said capture agents are specific antibodies, VHHs and/or antibody fragments.

The invention further provides a method for determining whether a cartilage sample is a sample of articular cartilage or growth plate cartilage, said method comprising determining in said cartilage sample the expression level of the gene expression products of the genes Grem1, FRZb and DKK1.

The invention further provides a method for predicting the likelihood of a sample of cartilage tissue will undergo endochondral ossification when *implanted in vivo* by determining the levels of Grem1, FRZb and DKK1 gene expression products in said sample.

The invention also provides Grem1, FRZb and DKK1 proteins or an expression vector comprising a nucleic acid encoding Grem1, FRZb and DKK1 proteins for use in the treatment of a joint disease. Preferably, said proteins are recombinant proteins.

### DETAILED DESCRIPTION OF THE DRAWINGS

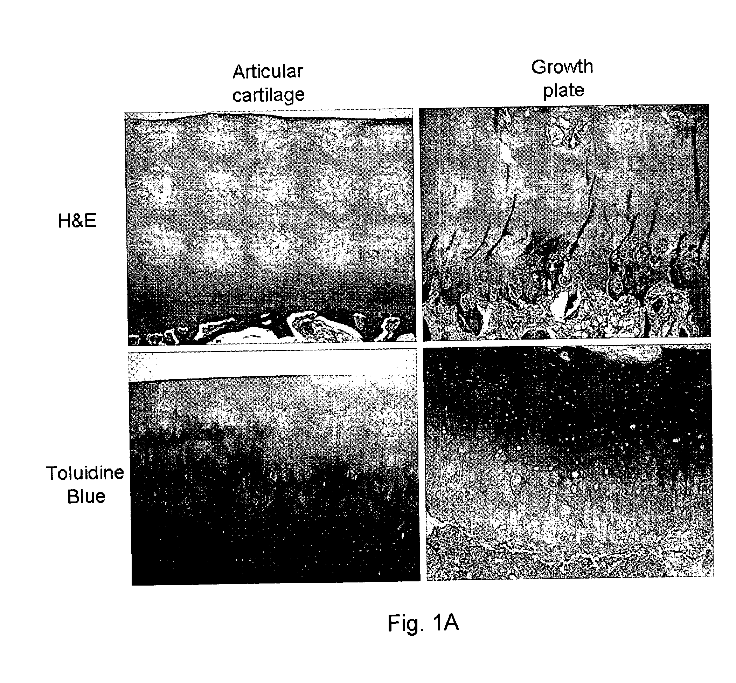

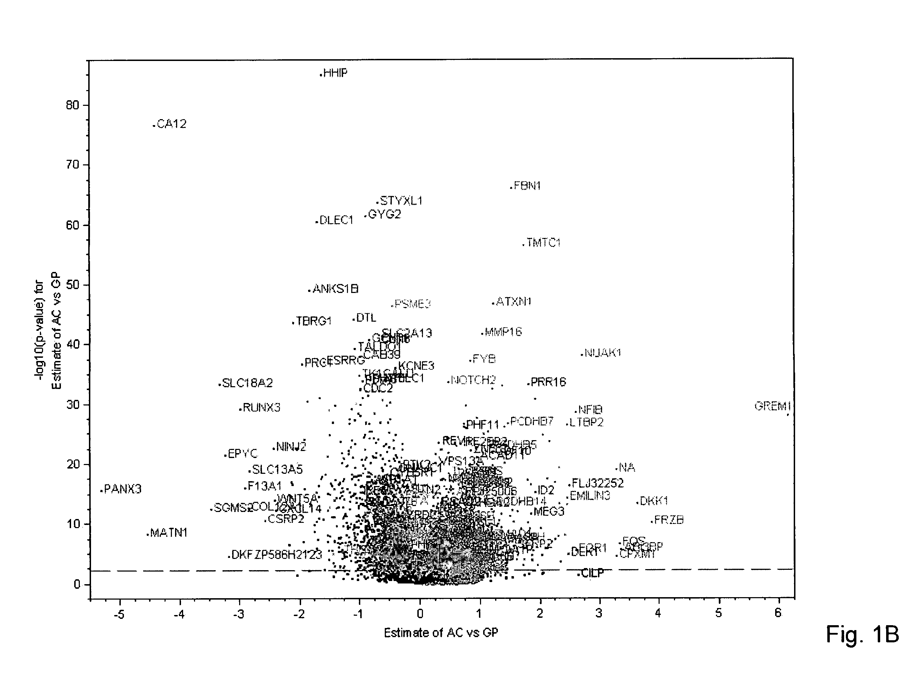

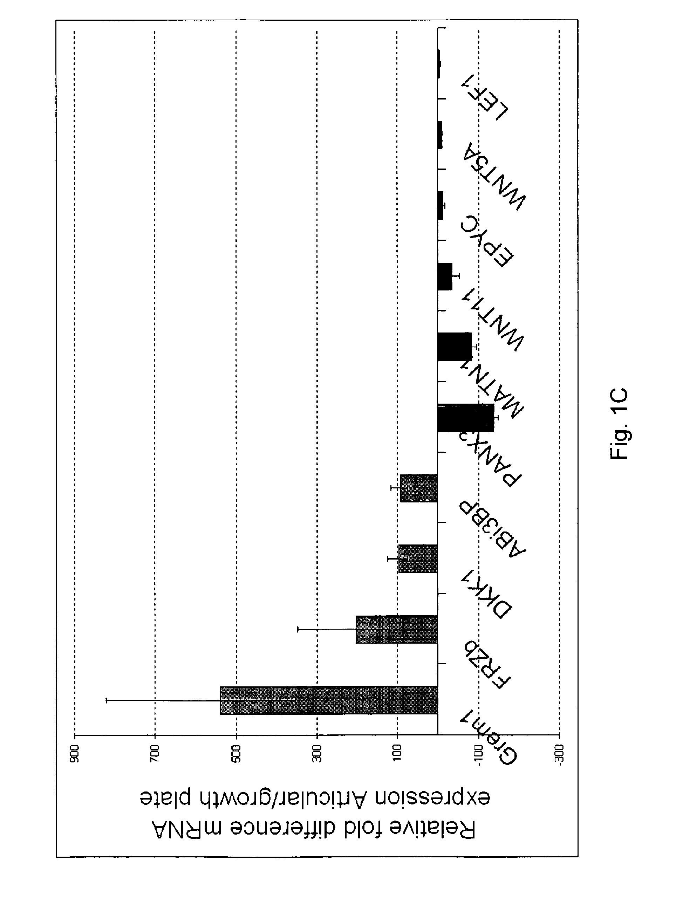

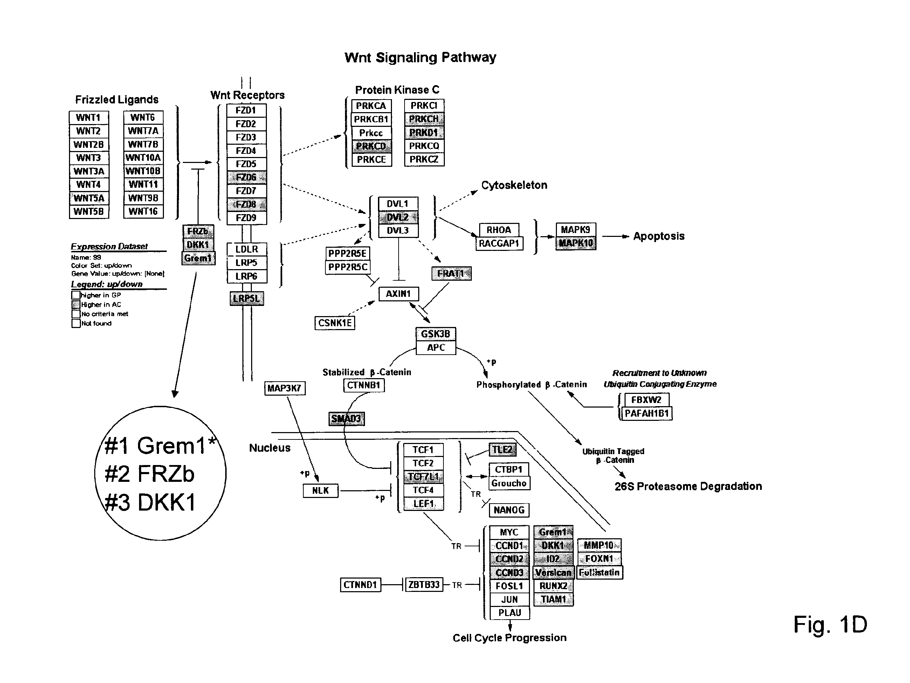

**Figure 1** shows that Grem1, FRZb and DKK1 are significantly more expressed in AC. RNA was isolated from collected growth plate (GP) and articular cartilages (AC) from patients with non-cartilage related diseases. The tissue samples were indeed unaffected as shown by histological analysis using toluidine blue, haematoxylin and eosin (A). Whole genome expression analysis was performed and 2915 genes were found to be significantly different expressed between AC and growth plate cartilage. The horizontal axis depicts the fold change and the Y-axis the p-value of significance. Dots represent genes. Relevant dots are identified by their gene symbol (B). Ten selected differentially expressed genes were successfully validated using quantitative PCR (qRT-PCR) (C). The top 3 most differentially regulated genes can antagonize Wnt-signaling (Grem1*, DKK1 and FRZb) and BMP-signaling (Grem1). Pathway analysis of differentially expressed genes showed mayor differences in Wnt signalling (Fig. 1 D) between AC and growth plate cartilage. *Although Grem1 officially is a BMP-antagonist it has been shown that it can inhibit Wnt via a currently unknown mechanism.

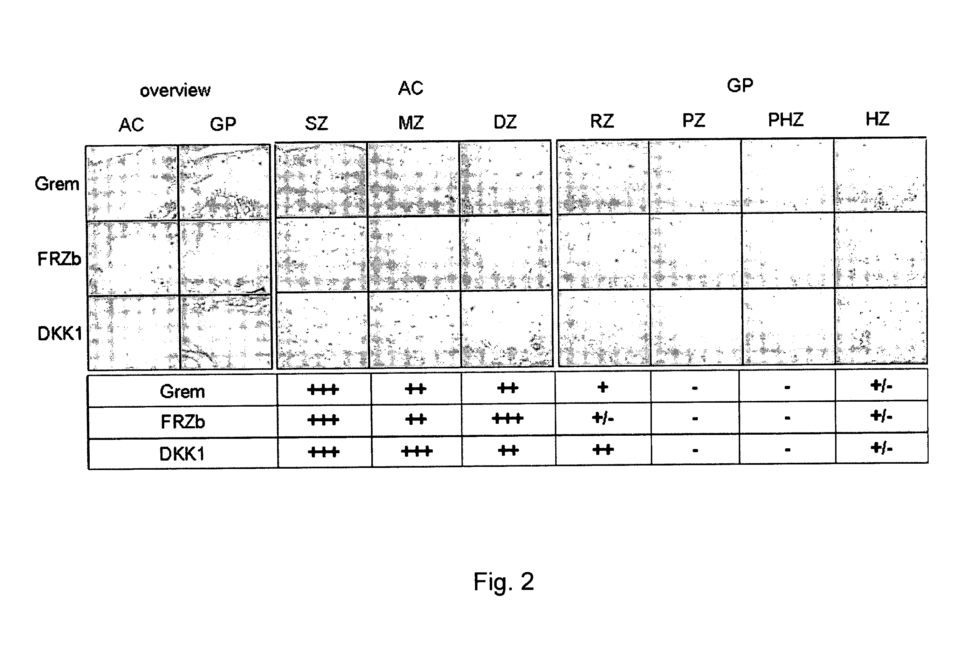

**Figure 2** shows the protein expression of Grem1, FRZb and DKK1 in AC and GP. Peroxidase immunohistochemistry of Grem1, FZRb and DKK1 on paraffin embedded sections of growth plate and articular cartilage showing a brown stain when positive. Left panel shows overview of the growth plate or articular cartilage, middle panel shows higher magnification images of different zones of the articular cartilage and the right panel shows higher magnification images of different zones of the growth plate. Abbreviations: SZ, superficial zone; MZ, middle zone; DZ, deep zone; RZ, resting zone; PZ, proliferative zone; PHZ, prehypertrophic zone; HZ hypertrophic zone.

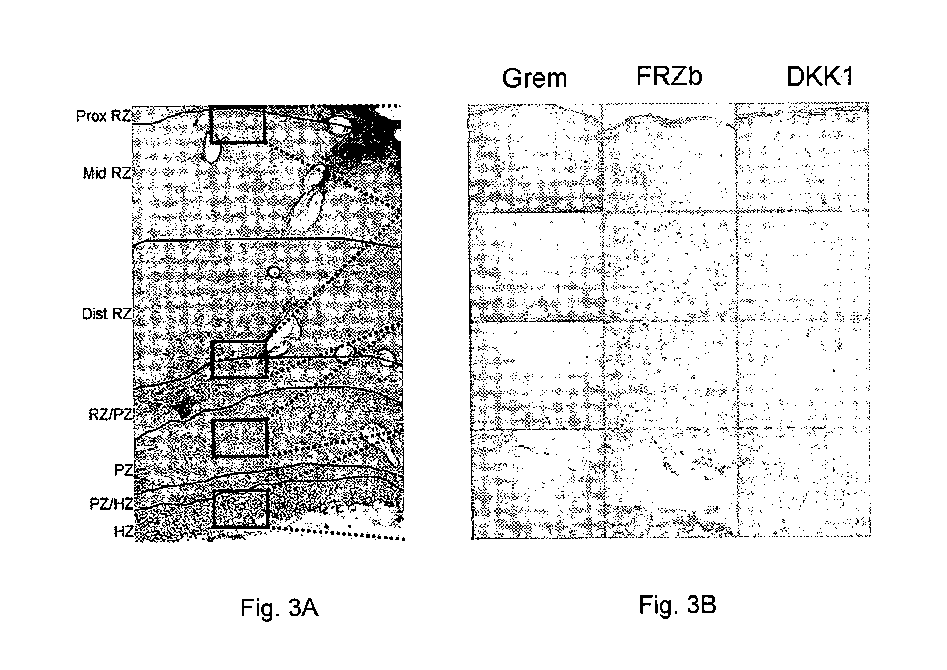

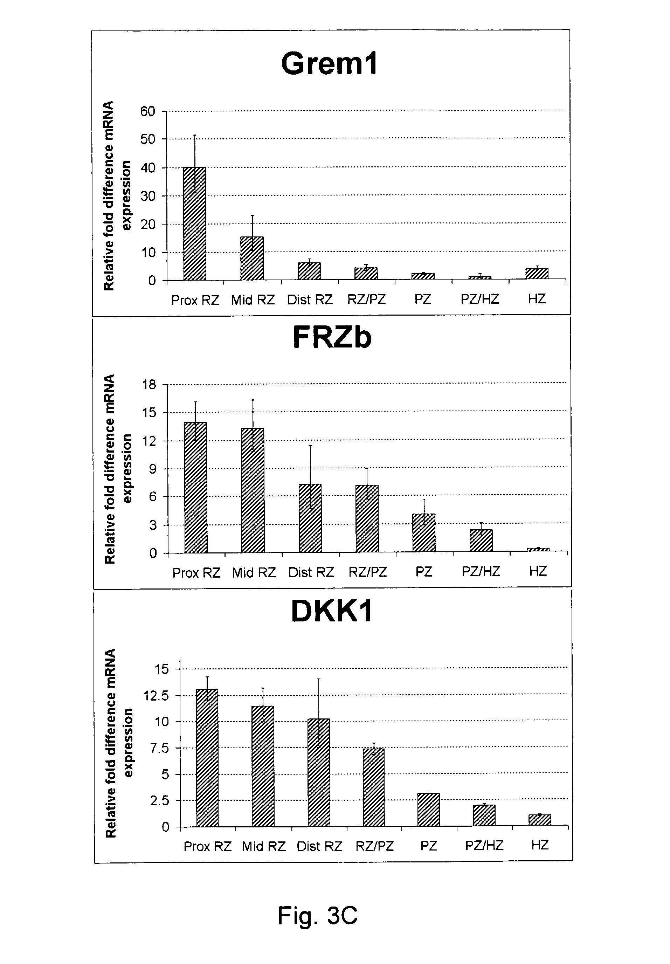

**Figure 3** shows the prenatal expression of Grem1, FRZb and DKK1. With appropriate consent two 20 week old fetuses with no known cartilage abnormalities, as confirmed with haematoxylin and eosin staining, were obtained (A). Peroxidase immunohistochemistry was performed for Grem1, FRZb and DKK1 (B). After the fetal femur was divided in 7 different zones, qRT-PCR was performed on each zone for Grem1, FRZb and DKK1(C). Abbreviations: Prox RZ, proximal resting zone; Mid RZ, middle resting zone; Dist RZ, distal resting zone; RZ, resting zone; PZ, proliferative zone; HZ, hypertrophic zone.

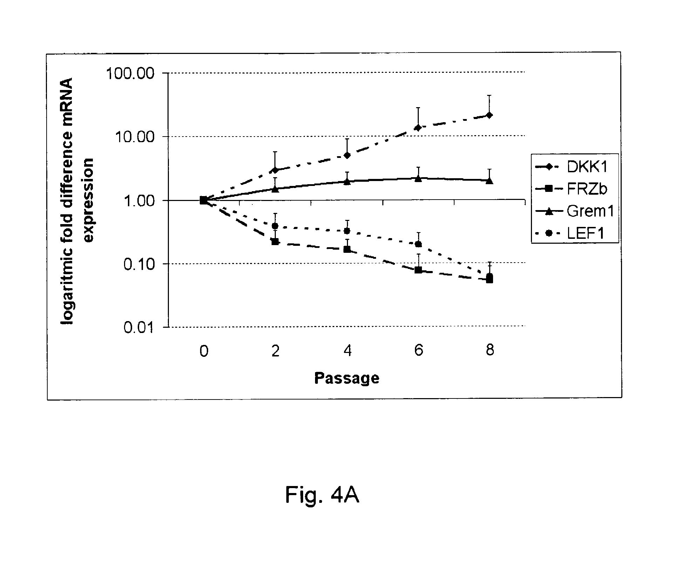

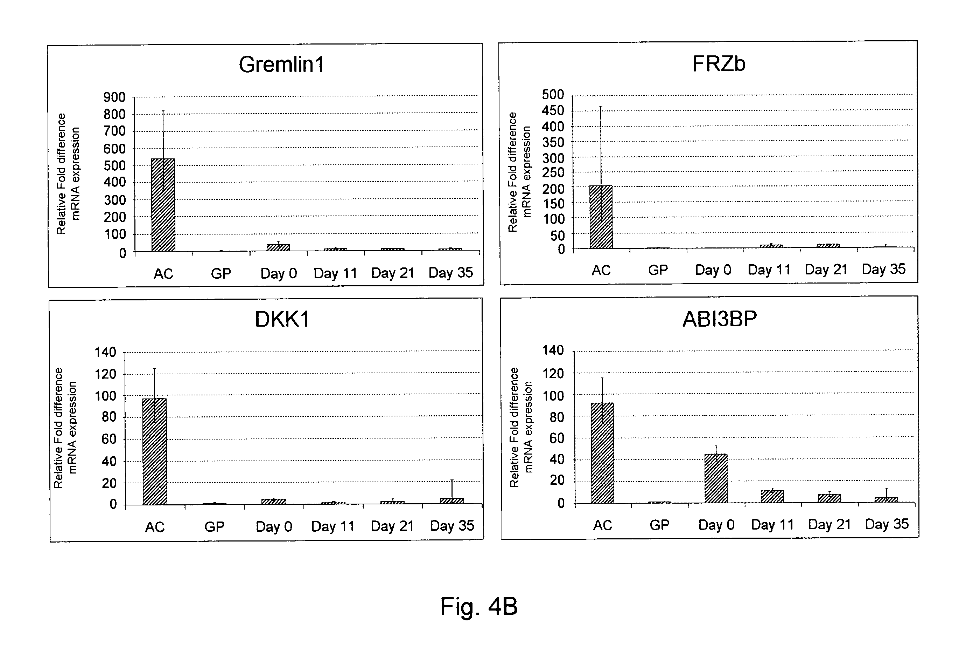

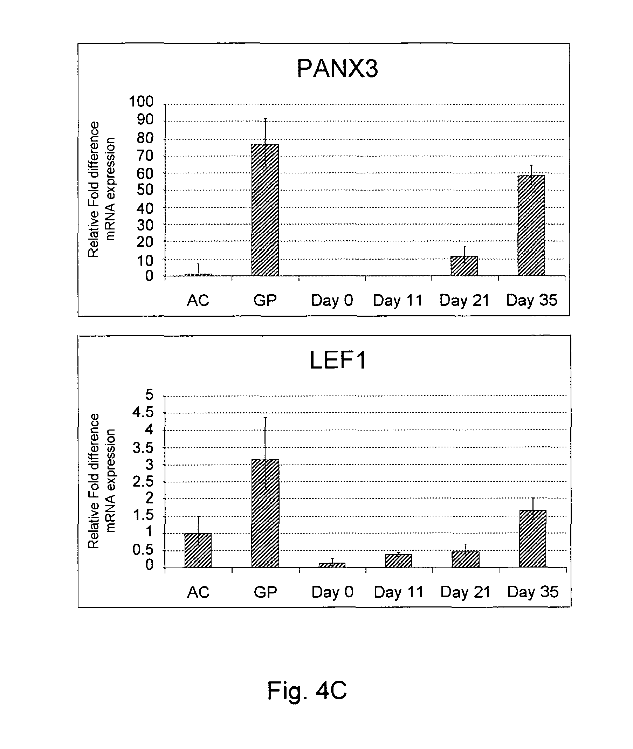

**Figure 4** shows the Grem1, FRZb and DKK1 expression of expanded chondrocytes and chondrogenically differentiating hMSC's. Human articular chondrocytes were isolated and expanded up to passage eight. Directly after isolation and every second passage RNA was isolated. qRT-PCR was used to analyze gene expression patterns of Grem1, FRZb and DKK1 during expansion (A). Chondrogenic differentiation of MSCs was induced in pellets from three different donors. RNA was isolated directly and after 11, 21 and 35 days. qRT-PCR analysis was performed on both articular enriched genes (B) and growth plate enriched genes (C) and compared with the normal expression in native articular and growth plate cartilage.

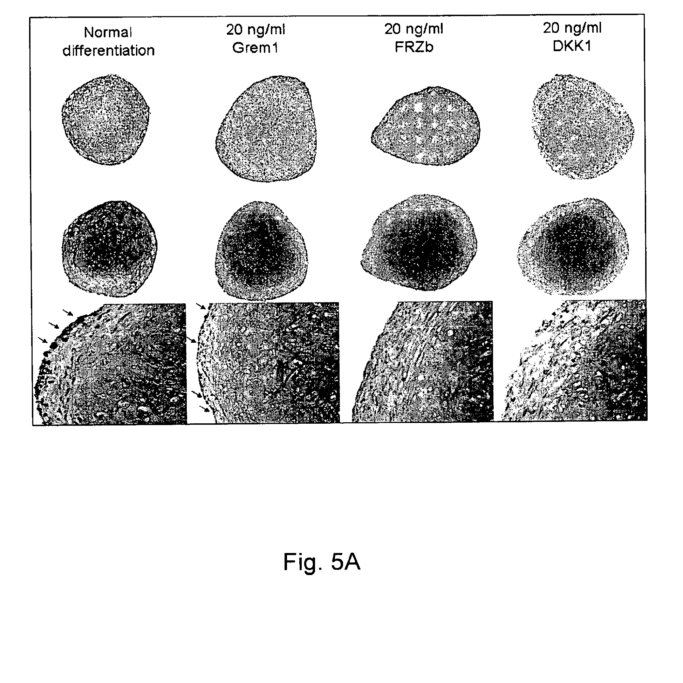

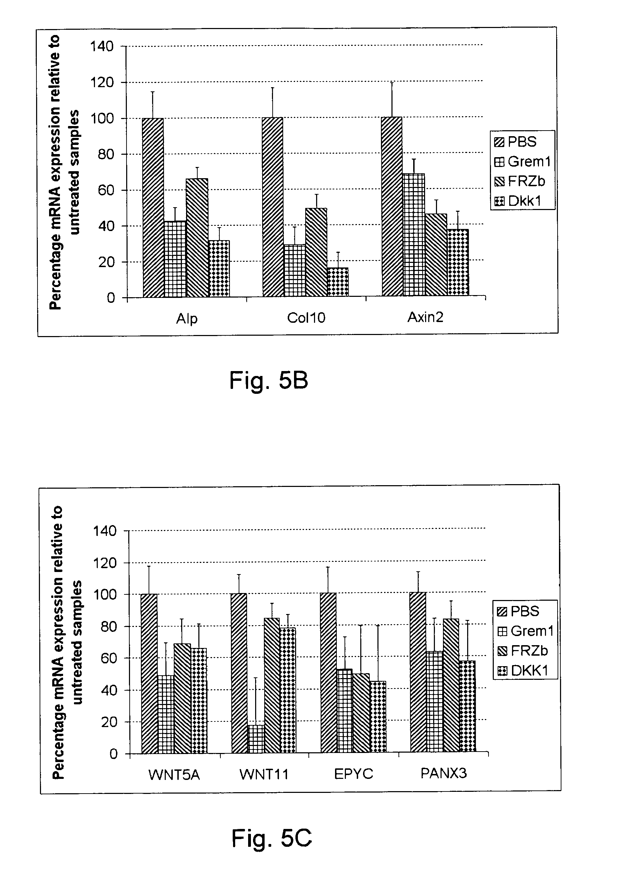

**Figure 5** shows that Grem1, FRZb and DKK1 inhibit hypertrophic differentiation. Chondrogenesis was induced in micromasses of hMSC's. After seven days they were treated with vehicle or recombinant proteins at a concentration of 20 ng/ml Grem1, FRZb or DKK1. After 35 days paraffin embedded sections were stained using haematoxylin combined with eosin or toluidine blue combined with Von Kossa (A). qRT-PCR analysis was performed on common hypertrophic markers alkaline phosphatase and collagen 10 as well as for direct Wnt target gene AXIN2 (B) and growth plate enriched genes Wnt5A, Wnt11, EPYC and PANX3 (C).

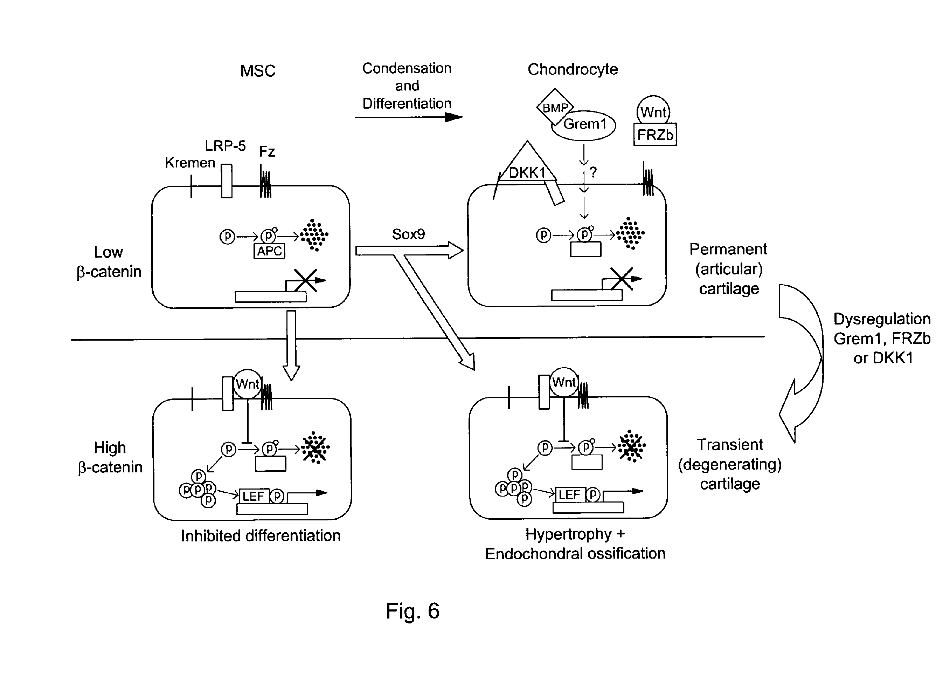

**Figure 6** shows a schematic representation of the hypothetic role of Grem1, FRZb and DKK1 in cartilage. Strong up regulation of Wnt signaling inhibits chondrogenesis. Under low levels of Wnt/β-catenin signaling chondrogenesis is allowed to occur, when Wnt/β-catenin is then activated it will lead to hypertrophic differentiation. However, sufficient expression of Grem1, FRZb and DKK1 is able to antagonize this hypertrophic differentiation, allowing the cartilage to retain its articular phenotype. Deregulation of Grem1, FRZb and DKK1 expression is associated with loss of the articular phenotype by hypertrophy and is associated with degenerative joint diseases.

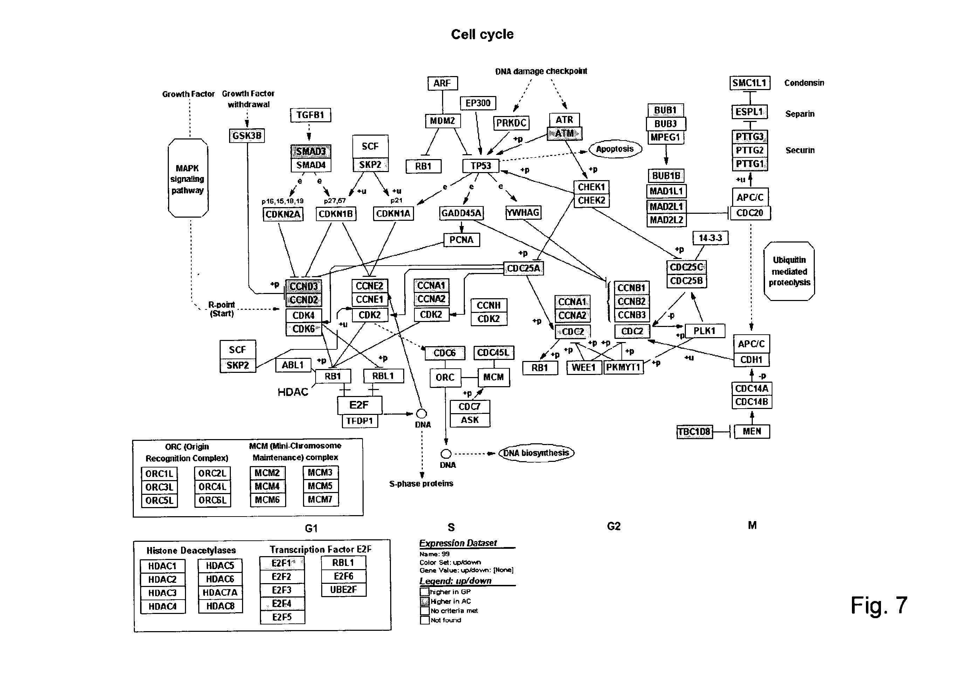

**Figure 7** shows a KEGG cell cycle pathway in which genes with differential expression between growth plate and articular cartilage as determined by microarray analysis are highlighted. Genes up regulated in growth plate are illustrated in green and genes overrepresented in articular cartilage are illustrated in red.

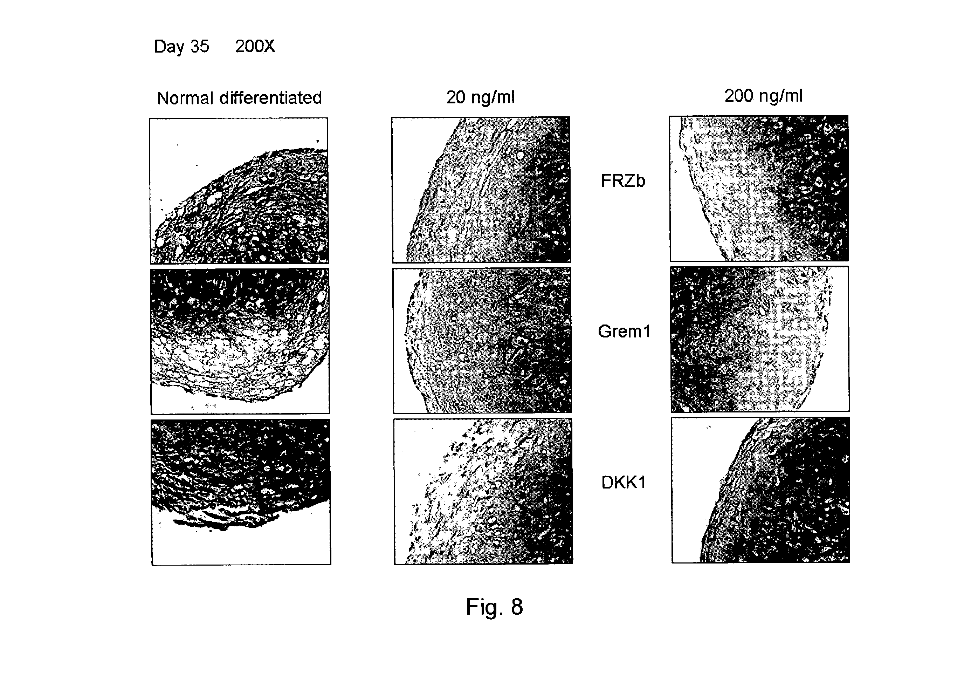

**Figure 8** shows that Grem1, FRZb and DKK1 inhibit hypertrophic differentiation. Chondrogenesis was induced in pellet cultures of hMSC's. After seven days they were treated with vehicle or recombinant proteins at a concentration of either 20 or 200 ng/ml Grem1, FRZb or DKK1. After 35 days paraffin embedded sections were stained using toluidine blue combined with Von Kossa.

### DETAILED DESCRIPTION

### Definitions

The term "sample obtained from an individual" as used herein refers to any sample comprising a gene expression product originating from said joint. Preferred samples include a sample comprising tissue of said joint, including cells originating from said joint or a fluid produced by cells residing in said joint, preferably synovial fluid.

Other preferred samples comprise the secreted gene expression products originating from a chondrocyte residing in a joint. Said secreted gene expression products are found in fluids stemming from a joint or in fluids which have passed through a joint. Subsequently, the gene expression products are secreted in urine, where said gene expression products can be detected. Preferred samples therefore include but are not limited to saliva, whole blood, serum, lymphoid fluid and urine.

The term "condition of a sample" as used herein refers to a status of said sample, which can be correlated to the condition of a joint in belonging to the individual from which the sample originated.

The term "healthy control individual" as used herein refers to an individual who is not suffering of any symptoms of a joint disease as defined herein and is not suspected of developing symptoms of said joint disease. Preferably, said healthy control individual is a randomly selected person. In certain embodiments, a healthy control individual is an individual who may be suffering from an unrelated disease. Preferably said healthy control individual refers to an individual who is not suffering of any symptoms of said joint disease, preferably as determined by a qualified person and preferably determined by the most sensitive detections methods currently available such as radiography or MRI.

The term" reference population" as used herein refers to a population of healthy control individuals.

The term "joint disease" as used herein refers to a disease wherein a joint is affected. The term joint disease covers all joint diseases from which vertebrates can suffer. Preferably, the joint disease encompasses two types of joint diseases: those in which inflammation is the principal set of signs or symptoms and joint diseases, non-inflammatory, in which inflammation may be present to some degree (as after an injury) but is not the essential feature. Arthritis is a generic term for inflammatory joint disease. Regardless of the cause, inflammation of the joints may cause pain, stiffness, swelling, and some redness of the skin around the joint. Effusion of fluid into the joint cavity is common, and examination of this fluid is often a valuable procedure for determining the nature of the disease. The inflammation may be of such a nature and of such severity as to destroy the joint cartilage and underlying bone and cause irreparable deformities. Adhesions between the articulating members are frequent in such cases, and the resulting fusion with loss of mobility is called ankylosis.

In some embodiments, inflammation restricted to the lining of a joint (the synovial membrane). This joint disease is referred to as synovitis. In other embodiments, said joint disease comprises arthralgias. These are simply pains in the joints. As ordinarily used, arthralgias are characterized by the lack of other accompanying evidence of arthritis. In another embodiment, said joint disease comprises rheumatism. The term rheumatism does not necessarily imply an inflammatory state, but refers to all manners of discomfort of the articular apparatus including the joints and also the bursas, ligaments, tendons, and tendon sheaths. In another embodiment, said joint disease comprises inflammation of the spine and joints, which is called spondylitis.

Bursitis is another joint disease. Bursitis is an inflammation of a synovial bursa, the lubricating sac located over a joint or between tendons and muscles or bones, is called bursitis (or bursal synovitis). Bursas sometimes are affected along with the joints and tendon sheaths in rheumatoid arthritis and gout. Infectious agents introduced by penetrating wounds or borne by the bloodstream also may result in bursitis, but this is unusual. The prepatellar bursa, located on the lower part of the kneecap, is especially subject to involvement in brucellosis (undulant fever).

The term joint disease also covers a group of diseases called infectious arthritis. In this disease, joints may be infected by many types of microorganisms (bacteria, fungi, viruses) and occasionally by animal parasites.

Rheumatoid arthritis and allied disorders are also joint diseases according to the invention. In several types of arthritis that resemble infectious joint disease, no causative agent has been isolated. Principal among these is rheumatoid arthritis. This disorder may appear at any age but is most usual in the fourth and fifth decades. A type that affects children is called juvenile rheumatoid arthritis. Rheumatoid arthritis typically affects the same joints on both sides of the body. Almost any movable joint can be involved, but the fingers, wrists, and knees are particularly susceptible. The joints are especially stiff when the affected person awakes. Rheumatoid arthritis is not only a disease of the joints; fatigue and anemia indicate that there is a more generalized systemic involvement. A slight fever may sometimes be present. Lesions also occur in sites outside the joints. Involvement of bursas, tendons, and tendon sheaths is an integral part of the disease. Approximately one of five affected persons has nodules in the subcutaneous tissue at the point of the elbow or elsewhere.

Degenerative joint diseases are also joint diseases. Osteoarthritis is a degenerative joint disease which is a ubiquitous disorder affecting all adults to a greater or lesser degree by the time they have reached middle age. The name osteoarthritis is a misnomer insofar as its suffix implies that the condition has an inherently inflammatory nature. For this reason it frequently is called degenerative joint disease, osteoarthrosis, or arthrosis deformans. When the spine is involved, the corresponding term is spondylosis. Unlike rheumatoid arthritis, osteoarthritis is not a systemic disease and rarely causes crippling deformities. In the majority of instances, the milder anatomical changes are not accompanied by appreciable symptoms. The changes are characterized by abrasive wearing away of the articular cartilage concurrent with a reshaping of adjacent ends of the bones. As a result, masses of newly proliferated bone (osteophytes) protrude from the margins of the joints.

Another joint disease is hypertrophic osteoarthropathy. In approximately 5 to 10 percent of persons who have primary tumours within the chest, the ends of the bones near the joints become enlarged and painful. New bone is formed in the periosteum, and only occasionally do abnormalities develop within the joints themselves. Just how the chest abnormality leads to hypertrophic osteoarthropathy (disease of bones and joints with abnormal growth of bone) is somewhat of a mystery, but there is reason to believe that the vagus nerve is involved, since the condition is usually relieved promptly by cutting the vagus. It is also relieved by removal of the tumour. In this disorder the tips of the fingers become club-shaped, a painless lesion that occurs in many other circumstances as well.

The clinical manifestations of osteoarthritis vary with the location and severity of the lesions. The most disabling form of the disorder occurs in the hip joint, where it is known as malum coxae senilis. Osteoarthritis of the hip, like that of other joints, is classified as primary or secondary. In secondary osteoarthritis the changes occur as a consequence of some antecedent structural or postural abnormality of the joint. In about half the cases, however, even rigorous examination fails to disclose such an abnormality; in these instances the osteoarthritis is called primary.

In a preferred embodiment, said joint disease is characterized by the symptom of hypertrophic differentiation of chondrocytes. More preferably, said disease is further characterized by pathogenic endochondral ossification, preferably following on said hypertrophic differentiation of chondrocytes.

As used herein, the term "oligonucleotide" refers to a single-stranded nucleic acid. Preferably, said oligonucleotide is between 5bp to 300bp in lenth. More preferably, the sequence-specific oligonucleotide is at least 15 to 20 nucleotides in length, although in some cases longer probes of at least 20 to 25 nucleotides will be desirable.

The term "significantly" or "statistically significant" refers to statistical significance and generally means a two standard deviation (SD) above normal, or higher, or below, or lower concentration of the gene expression product.

The term "Gremlin1" as used herein refers to the Gremlin gene or a gene expression product thereof, also known as Drm. The protein encoded by the Gremlin gene is a highly conserved 20.7-kDa, 184 amino acid glycoprotein part of the DAN family and is a cysteine knot-secreted protein. Gremlin1 has RefSeq genecode NM_013372.

The term "FRZb" as used herein refers to a gene or a gene expression product. The FRZb protein is a Wnt-binding protein especially important in embryonic development. It is a competitor for the cell-surface G-protein receptor of the Frizzled family. FRZb has RefSeq genecode NM_001463.

The term "DKK1" as used herein refers to the DKK1 gene or a gene expression product thereof. DKK1 has RefSeq genecode NP_036374 in humans and NP_034181 in mice. It is an inhibitor of signal transduction via the Wnt-pathway by blocking the interaction between LRP5 or LRP6 with a G-protein receptor of the Frizzled family.

The term 'VHH' refers to the single heavy chain variable domain antibodies devoid of light chains. Preferably a VHH is an antibody of the type that can be found in Camelidae or cartilaginous fish which are naturally devoid of light chains or to a synthetic and non immunized VHH which can be constructed accordingly. Each heavy chain comprises a variable region encoded by V-, D- and J exons. Said VHH may be a natural VHH antibody, preferably a Camelid antibody, or a recombinant protein comprising a heavy chain variable domain.

A 'V exon' in the context of the present invention describes a naturally occurring V coding sequence such as those found in Camelids. VHH exons may be derived from naturally occurring sources or they may be synthesised using methods familiar to those skilled in the art and described herein.

As used herein, the term 'antibody' refers to an immunoglobulin which may be derived from natural sources or synthetically produced, in whole or in part. The terms 'antibody' and 'immunoglobulin' are used synonymously throughout the specification unless indicated otherwise.

An 'antibody fragment' is a portion of a whole antibody which retains the ability to exhibit antigen binding activity. A preferred antibody fragment is a Fab fragment, a single chain antibody, an Fc fragment, a VH-fragment.

### Embodiments

The inventors have found that the levels of expression products of the genes Grem1, FRZb and DKK1 in a sample originating from a joint are strongly correlated with the condition of the joints. Osteoarthritis was associated with significantly changed levels of Grem1 (Tardif G et al. Osteoarthritis Cartilage.17:263-70. 2009)

Destabilization of the joint micro-environment has been reported for two SNP mutations in FRZb. These mutations have been associated with increased risk for osteoarthritis and total hip replacement (Loughlin J et al. Proc Natl Acad Sci U S A.101:9757-62. 2004). However, this association has been disputed in a more powerful two population-based study (Kerkhof JM et al. Radiographic Osteoarthritis Cartilage.16:1141-9. 2008).

DKK1 has been previously been described as a master regulator of joint remodeling based on the formation and resorption of bone by TNF-mediated expression of DKK1 and blockade of DKK1 (Diarra D et al. Nat Med.13:156-63. 2007). However, the actions of DKK1 in the joint is not limited to bone formation alone, when osteoarthritic cartilage was compared with cartilage of femoral neck fractures, DKK1 mRNA levels of the former were found to be elevated (Weng LH et al. Osteoarthritis Cartilage.17:933-43. 2009). Conversely, another study demonstrated a decreased concentration DKK1 in the serum of an osteoarthritic knee, while the serum of a knee with rheumatoid arthritis typically had elevated levels of DKK1 (Voorzanger-Rousselot N et al. Ann Rheum Dis.68:1513-4. 2009).

All these markers are directly or indirectly involved in the canonical Wnt pathway which is implicated in the development of degenerative joint disease. These markers are extracellular modulators of the Wnt-pathway. It is therefore unexpected that the combined levels of the gene expression products of the Grem1, FRZb and DKK1 genes are far better correlated with the condition of a sample originating from a joint, or the condition of a sample comprising the (preferably secreted) gene expression products of a chondrocyte residing in a joint of an individual, than with any of the levels of these genes alone. Particularly, since Grem1, FRZb and DKK1 have partly redundant activities and thus may compensate each other. Thus measurement of only 1 of these molecules does not provide information over the activity of the Wnt pathway in the joint without having information on the expression of the other molecules. This is surprising, as usually biomarkers based on genes in the same pathway tend to have little added value, as their activities are all a reflection of the activity of the canonical pathway as a whole. In addition, the combined measurement of Grem1, FRZb and DKK1 is more accurate compared to measurement of any of these biomarkers when determined individually.

The invention therefore provides a method for evaluating the condition of a sample the gene expression products of a cell, preferably a chondrocyte residing in a joint of an individual, comprising determining the gene expression levels of the genes from the group comprising Grem1, FRZb and DKK1 in said sample.

Preferably, said method further comprises determining whether the gene expression products are present at a level that is different when compared to the level of the same gene expression products of in a sample of a healthy control individual or in a sample of an unaffected joint (preferably the lateral) of the same individual.

The invention further provides a method for determining whether an individual has an increased risk of having a joint disease, or developing symptoms of a joint disease or developing symptoms associated with progression of said joint disease wherein said developing symptoms occurs within a time frame of 10 years after the completion of said method, wherein said symptoms develop within 10 years after the completion of said method, wherein said method comprises performing the method to the invention and further determining whether said condition is associated with an increased risk compared to the average risk of a reference population based on the comparison of the gene expression levels. This can be done using epidemiological methods well known in the art. Said method may of course also be used to monitor and/or predict the effectiveness of a treatment.

Preferably, said disease affecting a joint comprises osteoarthritis, arthritis or rheumatoid arthritis (RA). Gene expression products comprise nucleic acids and proteins. Nucleic acids in the invention preferably comprise RNA. The levels of the gene expression products may be determined separately for each different gene expression product or as a single measurement for more different gene expression products simultaneously. Preferably, the determination of the level of the gene expression products is performed for each different gene expression product separately, resulting in a separate measurement of the level of the gene expression product for each different gene expression product. This enables a more accurate comparison of levels of gene expression products with the levels of the same gene expression products in a sample of a control. Said sample of a control may be a sample from an unaffected joint (preferably the lateral) of the same individual. In another preferred embodiment, a control sample comprises a sample of a single individual or of multiple samples from a control group comprising of at least two individuals, preferably four individuals. In a preferred embodiment, said control group comprises at least 10, but preferably more healthy control individuals. Preferably, a database comprising the levels of gene expression products of said controls is used, wherein preferably the gene expression levels are classified based on sex and/or age. If the gene expression level is determined of a gene expression product from more than one individual, usually the median or mean level of these individuals is used for comparison.

Determination of the level of the gene expression products according to the methods of the invention may comprise the measurement of the amount of nucleic acids or of proteins. In a preferred embodiment of the invention, determination of the level of the gene expression products comprises determination of the amount of RNA, preferably mRNA. A level can be the absolute level or a relative level compared to the level of another mRNA. mRNA can be isolated from the samples by methods well known to those skilled in the art as described, e.g., in Ausubel et al., Current Protocols in Molecular Biology, Vol. 1, pp.4.1.1 -4.2.9 and 4.5.1-4.5.3, John Wiley & Sons, Inc. (1996). Methods for detecting the amount of mRNA are well known in the art and include, but are not limited to, northern blotting, reverse transcription PCR, real time quantitative PCR (qPCR) and other hybridization methods. The amount of mRNA is preferably determined by contacting the mRNAs with at least one sequence-specific oligonucleotide which hybridises to said mRNA. In a preferred embodiment said mRNA is determined with two sequence-specific oligonucleotides which hybridise to different sections of said mRNA. The sequence-specific oligonucleotides are preferably of sufficient length to specifically hybridize only to the RNA or to a cDNA prepared from said mRNA.

The sequence-specific oligonucleotides may also comprise non-specific nucleic acids. Such non-specific nucleic acids can be used for structural purposes, for example as an anchor to immobilise the oligonucleotides. The sequence-specific oligonucleotide can be labelled with one or more labelling moieties to permit detection of the hybridized probe/target polynucleotide complexes. Labelling moieties can include compositions that can be detected by spectroscopic, biochemical, photochemical, bioelectronic, immunochemical, and electrical optical or chemical means. Examples of labelling moieties include, but are not limited to, radioisotopes, e.g., 32P, 33P, 35S, chemiluminescent compounds, labelled binding proteins, heavy metal atoms, spectroscopic markers such as fluorescent markers and dyes, linked enzymes, mass spectrometry tags, and magnetic labels. Oligonucleotide arrays for mRNA or expression monitoring can be prepared and used according to techniques which are well known to those skilled in the art as described, e.g., in Lockhart et al., Nature Biotechnology, Vol. 14, pp. 1675-1680 (1996); McGaII et al., Proc. Natl. Acad. Scl. USA, Vol. 93, pp. 13555-13460 (1996); and U.S. Patent No. 6,040,138.

A preferred method for determining the amount of mRNA involves hybridization of labelled mRNA to an ordered array of sequence-specific oligonucleotides. Such a method allows the simultaneously determination of the mRNA amounts. The sequence-specific oligonucleotides utilized in this hybridization method typically are bound to a solid support. Examples of solid supports include, but are not limited to, membranes, filters, slides, paper, nylon, wafers, fibers, magnetic or nonmagnetic beads, gels, tubing, polymers, polyvinyl chloride dishes, etc. In a preferred embodiment, hybridization is performed on a Genechip Hybridization oven (Affymetrix, High Wycombe, UK). Preferably, the chip is scanned using a GeneChip scanner (Affymetrix, High Wycombe, UK).

According to a preferred embodiment of the invention, the level(s) of the gene expression products is determined by measuring the amount of protein. The term "protein" as used herein may be used synonymously with the term "polypeptide" or may refer to, in addition, a complex of two or more polypeptides which may be linked by bonds other than peptide bonds, for example, such polypeptides making up the protein may be linked by disulfide bonds. The term "protein" may also comprehend a family of polypeptides having identical amino acid sequences but different post-translational modifications, particularly as may be added when such proteins are expressed in eukaryotic hosts. These proteins can be either in their native form or they may be immunologically detectable fragments of the proteins resulting, for example, from proteolytic breakdown. By "immunologically detectable" is meant that the protein fragments comprise an epitope which is specifically recognized by e.g. mass spectrometry or antibody reagents as described below. Proteins levels can be determined by methods known to the skilled person, comprising but not limited to: mass spectrometry, Western blotting, immunoassays, protein expression assay, protein microarray, ELISA, RIA, IRMA or other techniques for quantitative measurement of protein concentrations in patient samples well known to an expert in the field.

A preferred embodiment of the invention provides a protein microarray (Templin et al. 2004; Comb.Chem.High Throughput Screen., vol. 7, no. 3, pp. 223-229) for simultaneous binding and quantification of the at least two biomarker proteins according to the invention. The protein microarray consists of molecules (capture agents) bound to a defined spot position on a support material. The array is then exposed to a complex protein sample. Capture agents such as antibodies are able to bind the protein of interest from the biological sample. The binding of the specific analyte proteins to the individual spots can then be monitored by quantifying the signal generated by each spot (MacBeath 2002; Nat. Genet, vol. 32 Suppl, pp. 526-532; Zhu & Snyder 2003; Curr.Opin.Chem.Biol., vol. 7, no. 1, pp. 55-63). Protein microarrays can be classified into two major categories according to their applications. These are defined as protein expression microarrays, and protein function microarrays (Kodadek 2001 ; Chem.Biol., vol. 8, no. 2, pp. 105-115). Protein expression microarrays mainly serve as an analytic tool, and can be used to detect and quantify proteins, antigen or antibodies in a biological fluid or sample. Protein function microarrays on the other hand can be used to study protein-protein, enzyme-substrate and small molecule-protein interactions (Huang 2003; Front Biosci., vol. 8, p. d559-d576). Protein microarrays also come in many structural forms. These include two-dimensional microarrays constructed on a planar surface, and three-dimensional microarrays which use a Flow- through support.

Types of protein microarray set-ups: reverse phase arrays (RPAs) and forward phase arrays (FPAs) (Liotta et al. 2003; Cancer Cell, vol. 3, no. 4, pp. 317-325). In RPAs a small amount of a tissue or cell sample is immobilized on each array spot, such that an array is composed of different patient samples or cellular lysates. In the RPA format, each array is incubated with one detection protein (e.g., antibody), and a single analyte endpoint is measured and directly compared across multiple samples. In FPAs capture agents, usually an antibody or antigen, are immobilized onto the surface and act as a capture molecule. Each spot contains one type of immobilized antibody or capture protein. Each array is incubated with one test sample, and multiple analytes are measured at once.

One of the most common forms of FPAs is an antibody microarray. Antibody microarrays can be produced in two forms, either by a sandwich assay or by direct labelling approach. The sandwich assay approach utilizes two different antibodies that recognize two different epitopes on the target protein. One antibody is immobilized on a solid support and captures its target molecule from the biological sample. Using the appropriate detection system, the labelled second antibody detects the bound targets. The main advantage of the sandwich assay is its high specificity and sensitivity (Templin, Stoll, Bachmann, & Joos 2004; Comb. Chem. High Throughput Screen., vol. 7, no. 3, pp. 223-229). High sensitivity is achieved by a dramatic reduction of background yielding a high signal-to noise ratio. In addition, only minimal amounts of labelled detection antibodies are applied in contrast to the direct labelling approach were a huge amount of labelled proteins are present in a sample. The sandwich immunoassay format can also be easily amenable to the field of microarray technology, and such immunoassays can be applied to the protein microarray format to quantify proteins in conditioned media and/or patient sera (Huang et al. 2001 ; Clin.Chem.Lab Med., vol. 39, no. 3, pp. 209-214; Schweitzer et al. 2002; Nat Biotechnol., vol. 20, no. 4, pp. 359-365).

In the direct labelling approach, all proteins in a sample are labelled with a fluorophore. Labelled proteins that bind to the protein microarray such as to an antibody microarray are then directly detected by fluorescence. An adaptation of the direct labeling approach is described by Haab and co-workers (Haab, Dunham, & Brown 2001; Genome Biol., vol. 2, no. 2, p). In this approach, proteins from two different biological samples are labelled with either Cy3 or Cy5 fluorophores. These two labelled samples are then equally mixed together and applied to an antibody microarray. This approach, for example, allows comparisons to be made between diseased and healthy, or treated and untreated samples. Direct labelling has several advantages, one of which is that the direct labelling method only requires one specific antibody to perform an assay.

Miniaturized and multiplexed immunoassays may also used to screen a biological sample for the presence or absence of proteins such as antibodies (Joos et al. 2000; Electrophoresis, vol. 21, no. 13, pp. 2641-2650; Robinson et al. 2002; Nat. Med., vol. 8, no. 3, pp. 295-301).

In a preferred embodiment of the invention, the detection or capture agents such as the antibodies are immobilized on a solid support, such as for example on a polystyrene surface. In another most preferred embodiment, the detection or capture agents are spotted or immobilized in duplicate, triplicate or quadruplicate onto the bottom of one well of a 96 well plate.

There are various methods known for coating surfaces having affinity for (glyco)-proteins, in particular antibodies. Typically, these methods use a reactive group which binds covalently or non-covalently to an antibody. For example, slides coated with aminoproplylsilane are used for non-covalent adsorption of protein. Epoxysilane coated slides are reactive with lysine, arginine, cysteine and hydroxyls at pH 5-9. Aldehyde coated slides are reactive with lysine and arginine where pH 7-10 drives Schiffs base reaction. A skilled person will know how to select the right coating suitable for use in combination with the selected surface material and test the affinity for antibodies.

In a preferred embodiment, said solid support is a glass support coated with a synthetic or natural polymer with free reactive groups that can be used for functionalization and subsequent for covalent coupling of peptides, proteins and/or antibodies using a spotter. Free reactive groups comprise, but are not limited to amides (NH₂) (such as in polyamides), carboxyl-group (COOH), alcohol-group (OH) or sulfones (SH). These glasses are incubated with synovial fluid or serum. These glasses are preferably read out using surface plasma resonance (SPR), preferably of IBIS B.V. (the Netherlands). Antibodies or VHHs can be spotted on a sensing surface and covalently attached to this surface using techniques well known in the field. The appropriate sensing surface for generating a protein microarray depends on the read out method for example a gold surface for read out protein binding in surface plasma resonance (SPR), preferably of IBIS B.V. and is well known by experts in the field.

An expression level is classified as different when said expression level of said gene expression product is statistically significantly increased or decreased in said individual compared to the level of the same gene expression product found in control individuals. Preferably, an expression level is significantly increased or decreased if it is outside the 95% confidence interval, based on expression values in control individuals. Even more preferably, said expression level is outside the 99% confidence interval. Preferably, said confidence interval is based on expression levels in controls which are corrected for age and sex. In preferred embodiments, said difference is classified as statistically significant if the expression level is at least a 20 percent increased or decreased compared to expression level of the same gene expression product in control individuals. Preferably, the increase or decrease is at least 20, 25, 30, 35, 40, 45, 50, 75, 100, 150, 200 or 250 percent. Most preferably, said increase or decrease is at least 100 percent.

In another embodiment, the invention further provides a method according to the invention, further comprising a step of determining in a sample of said individual the level of gene expression products of at least one of the genes selected from the group LTBP1, LTBP2, ID2, ID3, GDF10, Smad3, TGFβ3, Wnt5A, Wnt10B, Wnt11, FZD6, FZD8, FZD10, LEF1, GSK3B, Co13A1, Co15A2, KAL1, Co16A3, FBN1, Co114A1, Matn1, Co112A1, Co14A5, MFAP3, FBN3, Co14A6, CPXM1, CILP, DLK1, SRPX2, CHRDL2, LTBP2, CFH, MMP2, Wnt5A and WFDC1 and/or a gene coding for an BMP antagonist or a Wnt antagonist are present at a level that is different when compared to the level of the same gene expression products of in a sample of a control. An advantage thereof is that the method according to the invention is even more accurate. These genes are well known in the art. The names of the gene followed by the REFSEQ Gene ID's are herewith provided. LTBP1: 4052, LTBP2: 4053, ID2: 3398, ID3: 3399, GDF10: 2662, Smad3: 4088, TGFβ3: 7043, Wnt5A: 7474, Wnt10B: 7480, Wnt11: 7481, FZD6: 8323, FZD8: 8325, FZD10: 11211, LEF1: 51176, GSK3B: 2932, Co13A1: 1281, Col5A2: 1290, KAL1: 3730, Co16A3: 1293, FBN1: 2200, Co114A1: 7373, Matn1: 4146, Col12A1: 1303, Co14A5: 1287, MFAP3: 4238, FBN3: 84467, Co14A6: 1288, CPXM1: 56265, CILP: 8483, DLK1: 8788, SRPX2: 27286, CHRDL2: 25884, LTBP2: 4053, CFH: 3075, MMP2: 4313, Wnt5A: 7474, WFDC1: 58189.

Antagonists of the BMP and Wnt pathway are very important for maintaining the homeostase of a joint. Therefore, determining the level of said antagonist improves the accuracy and the predictive value of the method according to the invention. Said gene coding for a BMP antagonist comprises [name of the gene followed by the REFSEQ Gene ID's] CHRD: (8646), NOG: 9241), CER1: 9350), DAND5: 199699), NBL1: 4681), GREM1: 26585), GREM2: 64388), SOST: 50964), SOSTDC1: 25928), TWSG1: 57045), and BMPER: 168667). Said gene coding for a Wnt antagonist comprises sFRP1 (6422), sFRP2 (6423), sFRP3 (=FRZb: 2487), sFRP4 (6424) , sFRP5 (6425), WIF-1 (11197), CER1: 9350 (which is also a BMP antagonist), DAND5: 199699 (also BMP antagonist), DKK1: 22943), DKK2: 27123), DKK3: 27122), DKK4: 27121), DKKL1: 27120); SOST: 50964 (which is also a BMP antagonist).

In another embodiment, the invention further provides a method according to the invention, further comprising a step of determining in a sample of said individual the level of gene expression products of at least one of the genes selected from the group LTBP1, LTBP2, ID2, ID3, GDF10, Smad3, TGFβ3, Wnt5A, Wnt10B, Wnt11, FZD6, FZD8, FZD10, LEF1, GSK3B, Col3A1, Col5A2, KAL1, Col6A3, FBN1, Col14A1, Matn1, Col12A1, Col4A5, MFAP3, FBN3 and Col4A6 are present at a level that is different when compared to the level of the same gene expression products of in a sample of a control.

In some embodiments, said at least one gene is selected from the BMP related molecules including LTBP1, LTBP2, ID2, ID3, GDF10, Smad3 and TGFβ3. In other preferred embodiments said at least one gene is selected from the Wnt related molecules such as Wnt5A, Wnt10B, Wnt11, FZD6, FZD8, FZD10, LEF1 and GSK3B. An advantage thereof is that the method according to the invention is more accurate. In other preferred embodiments, said at least one gene is selected from the group of Col3A1, Col5A2, KAL1, Col6A3, FBN1, Col14A1 Matn1, Col12A1. An advantage thereof is that the method according to the invention is more accurate. In another preferred embodiment, said at least one gene is selected from the group consisting of Matn1, Col12A1, Col4A5, MFAP3, FBN3 and Col4A6. An advantage thereof is that the method according to the invention is more accurate. Preferably said at least one gene comprises at least 2 genes, more preferably at least 3, 4, 5, 6, 7, 8, 9, 10, 15, 20, 25 or 27 genes. If more genes are used in the method, the method will generate less false positive and/or false negative results.

In a preferred embodiment, said disease affecting a joint comprises osteoarthritis, arthritis or rheumatoid arthritis (RA). An advantage thereof is that the biomarkers are more predictive for these diseases. Most preferably, said disease is osteoarthritis. An advantage thereof is that the biomarkers are more predictive for osteoarthritis.

Preferred samples include whole blood, serum, saliva, urine, skin, synovium, joint or bone biopsies and synovial fluids because these samples comprise relevant gene expression products. Preferably, said sample comprises a cell sample, because cell samples comprise proteins and nucleic acids. In some preferred embodiments, said sample comprises synovial fluid, because this contains a high amount of relevant gene expression products. In some preferred embodiments, the biological sample is a blood sample, because a blood sample is easy obtainable and comprises large amounts of relevant gene expression products.

In some preferred embodiments, the cell or cells comprised by the sample is a chondrocyte. An advantage thereof is that the expression levels of a chondrocyte are highly correlated with presence or progression of said joint disease. In some preferred embodiments, said chondrocyte is a growth plate chondrocyte, in other preferred embodiments an articular chondrocyte. In some embodiments said cell is present in articular cartilage. In some preferred embodiments said cell is present in the proximal zone of said articular cartilage. In some embodiments, said cell is preferably present in the proximal resting zone of the perichondrium of a bone. Preferably, said individual is a mammal, preferably a human.

In some preferred embodiments, said method according to the invention further comprises a step of determining another biochemical biomarker which is associated with a joint disease. Such markers are well known to a skilled person and are reviewed for example in W.E. van Spil et al, Osteoarthritis and Cartilege xxx (2010) 1-8. Preferably, said another biochemical marker comprises uCTX-II and/or sCOMP.

In a more preferred method according to the invention said time frame is 8, 6, 5, 4, 3 or 2 years. It is preferred that the method is used to determine the risk of developing said joint disease within shorter periods, because it limits the insecurity of a tested individual.

In another aspect, the invention relates to a kit of parts comprising up to 50 reagents, sequence specific oligonucleotides and/or capture agent for detecting Grem1, FRZb and DKK1.

More preferably, said kit further comprises at least one of the gene expression products of the genes LTBP1, LTBP2, ID2, ID3, GDF10, Smad3, TGFβ3, Wnt5A, Wnt10B, Wnt11, FZD6, FZD8, FZD10, LEF1, GSK3B, Col3A1, Co15A2, KAL1, Co16A3, FBN1, Col14A1, Matn1, Col12A1, Co14A5, MFAP3, FBN3, Co14A6, CPXM1, CILP, DLK1, SRPX2, CHRDL2, LTBP2, CFH, MMP2, Wnt5A and WFDC1. More preferably, said at least one of the gene expression products comprises the gene expression products of at least 10, 15, 20, 25, 30, 35, 40 or 45 genes.

In the present invention, the inventors report the differences between the genetic fingerprints of healthy human articular and growth plate cartilage effectively generating biomarkers able to discriminate between the two different hyaline cartilage subtypes. Amongst these biomarkers were the gene expression products of the genes Gremlin1, FRZb and DKK1, which had strongly enhanced levels of both RNA and protein in the articular cartilage. These differences were already present during fetal development as demonstrated in femurs from healthy fetal donors at 20 weeks of gestation with gene expression and immunohistochemical analysis.

With the panel of genes as defined herein, created by selection from both articular and growth plate cartilage enriched genes, the inventors can accurately distinguish between hyaline cartilage subtypes. The inventors used micro-array analysis to identified genetic fingerprints that are highly specific for the two types of hyaline cartilage, growth plate and articular cartilage, which can be used to optimize tissue engineering strategies to obtain articular cartilage. Comparison of the genetic fingerprints has identified the Wnt- and BMP-antagonists, FRZb, DKK1 and Grem1 as specific markers for articular cartilage, which are differentially expressed from an early age. The inventors show that these antagonists prevent hypertrophic chondrocyte differentiation. These data suggest that Wnt- and BMP-antagonists play a prominent role in establishing a joint micro-environment which prevents articular chondrocytes from undergoing endochondral ossification.

The invention therefore further provides a method for determining whether a cartilage sample is a sample of articular cartilage or growth plate cartilage, said method comprising determining in said cartilage sample the expression level of the gene expression products of the genes Grem1, FRZb and DKK1. This grants new possibilities to determine and tailor newly formed cartilage for tissue engineering strategies, be it articular-like for cartilage or growth plate-like for bone tissue engineering. Preferably, RNA levels of these genes is determined, preferably, using RT-qPCR. In another embodiment, said levels of Grem1, FRZb and DKK1 gene expression products is determined by using immunohistochemistry techniques using antibodies against Grem1, FRZb and DKK1 proteins. Preferably, said sample is stained using haematoxylin and/or eosin.

### Grem1, FRZb and DKK1 proteins or expression vectors encoding Grem1, FRZb and DKK1 proteins for use in the treatment of a joint disease

Chondrogenically differentiating MSCs resemble more growth plate cartilage rather than articular cartilage. Moreover, once implanted such in vitro generated cartilage readily undergoes endochondral ossification, while identically treated isolated articular chondrocytes are resilient to this process (Jukes JM, et al. Proc Natl Acad Sci U S A.105:6840-5. 2008). In the present invention it is demonstrated that expanded articular chondrocytes have continued expression of the antagonists Grem1, FRZb and DKK1 even during extensive expansion, and that chondrogenesis of hMSC's does not induce the expression of these genes, potentially explaining their endochondral ossification. In addition, supplementation of Grem1, FRZb and DKK1 guides differentiating hMSC's to a more articular cartilage fingerprint in vitro. Due to the soluble nature of these antagonists it is considered that co-culturing of articular chondrocytes not only induces enhanced chondrogenesis (Hendriks J et al. J Tissue Eng Regen Med.1:170-8. 2007) but also limits hypertrophy and subsequent endochondral ossification.

Proper expression of Grem1, FRZb and DKK1 maintain Wnt/β-catenin signals sufficiently low to prevent hypertrophic differentiation, while allowing chondrogenesis allowing the build up and maintenance of permanent cartilage, such as articular cartilage. Dysregulation of Grem1, FRZb and DKK1 will lead to degeneration and therefore transient cartilage as depicted schematically in figure 6.

Addition of Gremlin1, FRZb or DKK1 to chondrogenically differentiating human mesenchymal stem cells (hMSCs), which are prone to undergo hypertrophic differentiation, led to reduced or even inhibited hypertrophic differentiation, while still obtaining a clear chondrogenic phenotype. Together, this strongly suggests a prominent role for Gremlin1, FRZb and DKK1 in establishing a joint micro-environment that prevents articular chondrocytes from undergoing hypertrophic differentiation and subsequent endochondral ossification.

The invention therefore further provides Grem1, FRZb and DKK1 proteins or expression vectors comprising a nucleic acid encoding Grem1, FRZb and DKK1 proteins for use in the treatment of a joint disease. Preferably, said proteins are recombinant proteins. Any expression vector which is suitable for medical use may be used. Preferred vectors are retroviral vectors. The medical use in the treatment of a joint disease of miRNAs, which downregulate expression of the Grem1, FRZb and DKK1 genes is also encompassed by the invention. Preferably said disease is characterized by pathogenic hypertrophy of chondrocytes, because the Grem1, FRZb and DKK1 gene expression products are most effective in reducing and/or preventing said hypertrophy, as shown in the experimental evidence. Preferably, said use is for the treatment of osteoarthritis and/or hypertrophic osteoarthropathy.

In a preferred embodiment, said medical treatment is preceded by a method for determining whether an individual has an increased risk of having a joint disease, of developing symptoms of a joint disease or of developing symptoms associated with progression of said joint disease according to the invention.

### Pharmaceutical Compositions and Therapeutic Uses

Furthermore, the invention provides pharmaceutical compositions allowing treatment of a joint disease comprising one or a combination of compounds indicated herein together with a pharmaceutically acceptable carrier, diluent or excipient are also provided herein. Pharmaceutical compositions can comprise polypeptides, polynucleotides or expression vectors according to the invention, collectively referred to as pharmaceutical compounds. The pharmaceutical compositions will comprise a therapeutically effective amount of a pharmaceutical compound as described herein.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician.

For purposes of the present invention, an effective dose will be from about 0.01 mg/ kg to 50 mg/kg or 0.05 mg/kg to about 10 mg/kg of the polynucleotide or polypeptide compositions in the individual to which it is administered.

A pharmaceutical composition can also contain a pharmaceutically acceptable carrier. The term "pharmaceutically acceptable carrier" refers to a carrier for administration of a therapeutic agent, such as a polypeptide, polynucleotide, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulphates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

### Delivery Methods

Once formulated, the pharmaceutical compositions of the invention can be (1) administered directly to the subject; (2) delivered *ex vivo,* to cells derived from the subject; or (3) delivered in vitro for expression of recombinant proteins.

Direct delivery of the compositions will generally be accomplished by injection, either subcutaneously, intraperitoneally, intravenously or intramuscularly, or delivered to the interstitial space of a tissue. The compositions can also be administered into the nervous system. Other modes of administration include topical, oral, suppositories, and transdermal applications, needles, and particle guns or hyposprays. Dosage treatment may be a single dose schedule or a multiple dose schedule.

Methods for the *ex vivo* delivery and re-implantation of transformed cells into a subject are known in the art and described in e.g., International Publication No. WO 93/14778. Examples of cells useful in *ex vivo* applications include, for example, stem cells, particularly hematopoietic, lymph cells, macrophages, dendritic cells, chondrocytes or tumor cells.

Generally, delivery of nucleic acids for both *ex vivo and in vitro* applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Various methods are used to administer the therapeutic composition directly to a specific site in the body. Receptor-mediated targeted delivery of therapeutic compositions containing an antisense polynucleotide, subgenomic polynucleotides, or antibodies to specific tissues is also used. Receptor-mediated DNA delivery techniques are described in, for example, Findeis et al., Trends in Biotechnol. (1993) 11:202-205; Wu et al., J. Biol. Chem. (1994) 269:542-46.

Pharmaceutical compositions containing polynucleotides are preferably administered in a range of about 100 ng to about 200 mg of polynucleotides for local administration in a gene therapy protocol. Concentration ranges of about 500 ng to about 50 mg, about 1 µg to about 2 mg, about 5 µg to about 500 µg, and about 20 µg to about 100 µg of polynucleotides can also be used during a gene therapy protocol. Factors such as method of action and efficacy of transformation and expression are considerations which will affect the dosage required for ultimate efficacy of the polynucleotides. Where greater expression is desired over a larger area of tissue, larger amounts of polynucleotides or the same amounts re-administered in a successive protocol of administrations, or several administrations to different adjacent or close tissue portions of, for example, a nerve ending or synaps, may be required to affect a positive therapeutic outcome. In all cases, routine experimentation in clinical trials will determine specific ranges for optimal therapeutic effect. A more complete description of gene therapy vectors, especially retroviral vectors, is contained in WO 98/00542, which is expressly incorporated herein.

The invention also provides a method for differentiating chondrocytes; preferably hMSC's to a more articular cartilage phenotype, preferably in vitro, by providing Grem1, FRZb and DKK1 gene expression products, preferably proteins.

The invention is further illustrated by the following examples. The examples are not to be interpreted as limiting the scope of the invention in any way.

### EXAMPLES

### Materials and Methods

### Origin samples

Four donors from which both growth plate and articular cartilage material was obtained, due to pathologies unrelated to the joint leading to amputation, e.g. osteosarcoma, were included for whole genome profiling. All samples were obtained from girls between the ages of 10 and 14 years old. The study was approved by the hospital ethical committees and informed consent was obtained. Samples were longitudinal cut with a bone saw and pieces were either directly frozen in liquid isopentane and stored at ―80°C or fixed in 10% formalin and subsequently embedded in paraffin.

### Total RNA extraction

After removal of the bone, the cartilage-samples were sectioned into 8 µm sections using a Reichert Jung 2055 microtome (Leica, Rijswijk, The Netherlands). Every fifth slide was stained with Haematoxylin to ensure that no bone contamination was present. Subsequently, the collected slices were homogenized in a 4M guanidine thiocyanate solution (Sigma). Total RNA isolation was performed by a method previously described by (Heinrichs C, et al. Endocrinology.135:1113-8. 1994). The recovered RNA was then purified using the RNAeasy kit (Qiagen) following the manufacturer's protocol. High quality RNA was confirmed by the Agilent 2100 bioanalyzer (Agilent).

RNA *from in vitro* cultures was obtained using the RNAeasy kit following manufacturer's protocol after being disaggregated using a 20 gauge syringe.

### Cell isolation and cultivation

MSCs were isolated from fresh bone marrow samples, obtained with written consent, as described previously ( Both SK et al. Tissue Eng.13:3-9. 2007). Briefly, aspirates were resuspended using a 20-gauge needle, plated at a density of 500,000 cells/cm² and cultured in hMSC proliferation medium containing a-MEM (Gibco), 10% heat-inactivated fetal bovine serum (Biowhittaker), 0.2 mM ascorbic acid (Sigma), 2 mM L-glutamine (Gibco), 100 U/ml penicillin with 100 mg/ml streptomycin (Gibco) and 1 ng/ml basic fibroblast growth factor (Instruchemie, Delfzijl, The Netherlands).

Human articular chondrocytes were isolated from femoral articular cartilage by collagenase digestion, after written consent was obtained. Isolated cells were seeded at 10000-200000 per cm² and proliferated in chondrocyte expansion medium containing DMEM (Gibco), 10% heat-inactivated fetal bovine serum (Biowhittaker), 1x non-essential amino acids, 0.2 mM ascorbic acid, 0.4 mM proline, 1% penicillin and streptomycin. Chondrocytes were expanded, taking RNA from every second passage RNA up to passage 8. All cells were grown in a humid atmosphere with 5% CO₂.

### Chondrogenic differentiation of hMSC's

To induce chondrogenic differentiation, hMSC's were seeded at 2.5x10⁵ in a U-shaped 96 well plates, centrifuged for 3 minutes at 2000 RPM and incubated at 37°C and 5% CO₂. Cells were cultured in chondrogenic medium composed of DMEM high glucose with 0.1 µM dexamethason (Sigma), 100 µg/ml sodium pyruvate (Sigma), 0.2 mM ascorbic acid, 50 mg/ml insulin-transferrin-selenite (ITS+Premix, BD biosciences), 100 U/ml penicillin, 100 µg/ml streptomycin, 10 ng/ml transforming growth factor β3 (TGF-β3, R&D system) and 500 ng/ml bone morphogenetic protein 6 (BMP-6, R&D system) up to 35 days refreshing the medium three times a week. On day 0, 11, 21 and 35 ten pellet cultures were stopped, eight were pooled and RNA was isolated and two were fixed in 10% formalin and subsequently embedded in paraffin.

### Histology and immunohistochemistry

In paraffin embedded samples were cut into 5 µm sections, deparaffinized in xylene and dehydrated by treatment with graded ethanols. Sections were stained either with haematoxylin and eosin, toluidine blue or toluidine blue combined with Von Kossa according to standard procedures.

For immunohistochemistry sections were incubated with 1:100 diluted primary antibodies against either gremlin, FRZb or DKK1 (respectively: sc-28873, sc-13941 and sc-25516; Santa Cruz) and further developed using the rabbit ABC staining system (sc-2018; Santa Cruz) according to manufacturers protocol.

### Microarray processing

Biotinylated antisense cRNA was prepared from the isolated RNA of in total thirteen specimens, eight articular and five growth plates, according to the Affymetrix standard labelling protocol. Afterwards, the hybridization on the chip was performed on a GeneChip Hybridization oven 640, then dyed in the GeneChip Fluidics Station 450 and thereafter scanned with a GeneChip Scanner 3000. All used equipment was from the Affymetrix-Company (Affymetrix, High Wycombe, UK).

### Statistical analysis

Gene expression profiling was performed using arrays of HG-U133_Plus_2-type from Affymetrix (Affymetrix, High Wycombe, UK). A Custom CDF Version 11 with Entrez based gene definitions was used to annotate the arrays. The Raw fluorescence intensity values were normalized applying quantile normalization. Differential gene expression was analysed based on loglinear mixed model ANOVA, using a commercial software package SAS JMP7 Genomics, version 3.1, from SAS (SAS Institute, Cary, NC, USA). A false positive rate of a=0.05 with Holm correction was taken as the level of significance. Principal Component Analysis (PCA) with Pearson product-moment correlation was performed to computes correlations between the expression of growth plates and articular cartilages.

### Pathway Analysis

The overrepresentation analysis (ORA) is a microarray data analysis that uses predefined gene sets to identify a significant overrepresentation of genes in data sets (Subramanian et al, 2005; Manoli et al, 2006). Pathways belonging to various cell functions such as cell cycle or apoptosis were obtained from public external databases (KEGG, http://www.genome.jp/kegg/). A Fisher's exact test was performed to detect the significantly regulated pathways.

P-values smaller than 0.05 were considered as significant. The genes were classified on functional clusters and pathway analysis was performed using Gene Set Enrichment Analysis (GSEA, http://www.broad.mit.edu/gsea/).] The raw and normalized data are deposited in the Gene Expression Omnibus database (http://www.ncbi.nlm.nih.gov/geo/; accession No. GSE-XXXXX). Roy J (2007) SAS for mixed models. In J Biopharm Stat, Littell RC, Milliken GA, Stroup WW, Wolfinger RD, Schabenberger O (eds), 2nd edn, Vol. 17, pp 363-365 | Article |

Subramanian A, Tamayo P, Mootha VK, et al. Gene set enrichment analysis: a knowledge-based approach for interpreting genome-wide expression profiles. Proc Natl Acad Sci U S A. 2005;102:15545-15550. [Abstract/Free Full Text] Manoli T, Gretz N, Grone HJ, Kenzelmann M, Eils R, Brors B. Group testing for pathway analysis improves comparability of different microarray datasets. Bioinformatics. 2006;22:2500-2506.[Abstract/Free Full Text]

### Quantitative real-time reverse transcriptase-polymerase chain reaction (qRT PCR)

MRNA levels from total RNA extracted from biopsies were determined by qRT-PCR. For this purpose, 1 µg of total RNA was reverse-transcribed using the iScript^{tm} cDNA synthesis kit (BioRad) in accordance with the manufacturer's instructions. 20 ng cDNA was amplified using iQ SYBR Green Supermix (BioRad, Hercules, CA, USA) on a MyIQ single color Real-time PCR detection system (BioRad) and analyzed using iQ^{tm}5 optical system software (Biorad). The qPCR amplifications were run under the following conditions: initial denaturation for 5 minutes at 95°C, then cycled 45 times at 95 °C for 15 seconds, 60°C for 30 seconds and 72 °C for 30 seconds and was followed by a melting curve. Final products were run on a 1% agarose gel confirming single bands of the appropriate sizes. Sequences of primers used are listed in supplement table 1

### Results

### Differential gene regulation between articular and growth plate cartilage

Macroscopical and histological examination, using haematoxylin and eosin, confirmed healthy and unaffected nature of all specimens (figure 1A). The isolated RNA was subjected to whole genome profiling in which articular was compared with growth plate cartilage specimens. In total 2195 genes were found to be significantly differentially regulated, of which 418 genes more than twofold (figure 1B, Figure 8). Ten genes of interest were selected and verified using RT-qPCR (figure 1C).

Interestingly, the three most differentially expressed genes, being highly enriched in the articular cartilage, are the Wnt- and BMP antagonists: FRZb, DKK1 and Gremlin1. Besides Grem1, several other BMP related molecules including LTBP1, LTBP2, ID2, ID3, GDF10, Smad3 and TGFβ3 were significantly higher expressed in articular chondrocytes. Wnt related molecules such as Wnt5A, Wnt10B, Wnt11, FZD6, FZD8, FZD10, LEF1 and GSK3B were as well differently regulated (Figure 1D). As to be expected, many cell cycle factors were transcribed at significantly higher levels in the growth plate than in articular cartilage (Figure 7).

In addition to these differences in signalling pathways, the expression of extracellular matrix molecules was as well dissimilar with Co13A1, Co15A2, KAL1, Col6A3, FBN1 and Col14A1 being higher expressed in articular chondrocytes and Matn1, Col12A1, Col4A5, MFAP3, FBN3 and Col4A6 expressed higher in growth plate chondrocytes.

Supplement table 1: Sequence of primers used in the described experiments. All primers are designed for SYBR green based qRT-PCR.

| Gene | orientation | 5' to 3' sequence |
|---|---|---|
| | For | AAAGCTCCACTGGGAGGAGT |
| ABl3BP | Rev | GTGGGTCGTGCTGAGATTTT |
| Alkaline | For | GACCCTTGACCCCCACAAT |
| Phsophatase | Rev | GCTCGTACTGCATGTCCCCT |
| | For | AGTGTGAGGTCCACGGAAAC |
| Axin2 | Rev | CTGGTGCAAAGACATAGCCA |
| | For | GCAACTAAGGGCCTCAATGG |
| Col10A1 | Rev | CTCAGGCATGACTGCTTGAC |
| | For | AGTACTGCGCTAGTCCCACC |
| DKK1 | Rev | TCCTCAATTTCTCCTCGGAA |
| | For | GCAGGACTTGTTCTGGGACT |
| EPYC | Rev | TCTCTGTTCCCTGAAGGCAT |
| | For | ACGGGACACTGTCAACCTCT |
| FRZb | Rev | CGAGTCGATCCTTCCACTTC |
| | For | CGCTCTCTGCTCCTCCTGTT |
| GAPDH | Rev | CCATGGTGTCTGAGCGATGT |
| | For | GTCACACTCAACTGCCCTGA |
| Grem1 | Rev | GGTGAGGTGGGTTTCTGGTA |
| | For | GGAAGGCGATTTAGCTGACA |
| LEF1 | Rev | GTGTTCTCTGGCCTTGTCGT |
| | For | TCCTCTCTGGCACTAGCCTC |
| MATN1 | Rev | CGAGAGCTGTCGACAACAAA |
| | For | GGGACTCACTGCTTCACCAT |
| PANX3 | rev | AACAGCAGATCGGAGCTGAG |
| | For | ATCAATTCCGACATCGAAGG |
| Wnt5A | Rev | CGTTCACCACCCCTGCT |
| | For | GCTCCTCCTGGGTGTGAC |
| Wnt11 | Rev | GATGGTGTCTTGGACAGCG |

### Protein expression of Gremlin, FRZb and DKK1 is predominantly in the articular cartilage and resting zone of the growth plate.

The translational differences corroborated with the transcriptional differences as demonstrated with peroxidase based immunohistochemistry, which showed a stronger staining of all three antagonists in the articular cartilage compared to the growth plate cartilage (figure 2). In contrast to the articular cartilage, the antagonists were not expressed throughout all the different zones in the growth plate. More precisely, Gremlin1, FRZb and DKK1 were present in the hypertrophic zone and more so in the resting zone, but were not detectable in the proliferative and the prehypertrophic zone.

### Differential expression of Grem1, FRZb, DKK1 in primary growth plates of a human foetal femur.

We hypothized that the difference in antagonists expression was not only limited to the postnatal state, when the secondary centre of ossification physically separates the articular cartilage from the growth plate, but also at earlier time points. The healthy and unaffected nature of the femurs of two human foetal donors, with a gestation of twenty weeks, was confirmed with toluidine blue as well as haematoxylin (Figure 3A). Immunohistochemistry revealed that gremlin predominantly resided at the proximal resting zone, which is the region destined to become the articular cartilage (Figure 3B). Although some gremlin could still be detected down to the distal resting zone, the staining is much less intense. Moreover, almost no staining could be detected in the proliferative and hypertrophic zones. FRZb was as well clearly detectable in the proximal resting zone and became less intense in more distal zones, and remained present at low levels in the proliferative zone and in the proximal hypertrophic zone. RNA isolated from seven different regions of the foetal femur was subjected to qPCR gene expression analysis and corroborated with the immunohistochemistry (figure 3C). Expression of both FRZb and DKK1 gradually declined from proximal resting zone to the hypertrophic zone 13.9 and 13.1 fold, respectively. Remarkably, grem1 RNA expression declined rapidly from proximal to distal resting zone. The decline continued up until the prehypertrophic zone and increased slightly again in the hypertrophic zone.

### Antagonists are persistently present in expanded chondrocytes

Next we investigated whether extensive expansion of isolated human chondrocytes would influence the expression levels of Grem1, FRZb and DKK1. Gene expression analysis indicated a decrease in FRZb that was counterbalanced by an almost equal increase in DKK1 resulting in a sustained inhibition on Wnt signalling. Moroever, the nuclear binding factor LEF1 is strongly decreased over time. The expression of Gremlin1 increases slightly but significantly over the period of eight passages (figure 4A).

### Chondrogenically differentiated hMSC's do not express Grem1, FRZb, DKK1 nor ABI3BP expression

Grem1, FRZb, DKK1 and ABI3BP are key discriminating factors between the transient growth plate and the permanent articular chondrocytes. We investigated whether chondrogenic differentiation of hMSC's would induce the expression of these antagonists. Both histological and gene expression analysis indicated chondrogenic differentiation. However, the expression of these antagonists were not induced during the differentiation, even more striking, both gremlin1 and ABI3BP were significantly down regulated. After 35 days of differentiation the MSC expressed all 4 genes at a level resembling the growth plate chondrocytes (figure 4B). Moreover, several growth plate enriched molecules, such as LEF1 and PANX3, are up regulated to levels which match with growth plate rather than articular chondrocyte levels (figure 4C).

### Addition of Grem1, FRZb or DKK1 during chondrogenic inhibits hypertrophic differentiation

Either 20 or 200 ng/µl recombinant human Grem1, FRZb and DKK1 were separately added to chondrogenically differentiating hMSC's to investigate whether these molecules have an inhibitory effect on hypertrophic differentiation. Histological analysis showed that all pellet cultures stained increasingly positive for glycosaminoglycans over time. Without the addition of antagonists mineralization was first observed around week three and was increased in week five, the mineralization was almost exclusively present in the periphery of the pellets. Addition of 20 ng/µl of recombinant antagonists lead to slightly lowered toluidine blue staining, but reduced mineralization with Grem1 strongly and with FRZb and DKK1 even to undetectable levels (Figure 5A). Addition of 200 ng/µl recombinant protein further reduced histological and morphological changes resembling hypertrophic differentiation (Figure 8). Addition of 200 ng/µl Grem1 reduced the mineralization to undetectable levels.

Expression of genes linked to hypertrophy such as alkaline phosphatase (ALP) and collagen 10 (Col10) were down regulated by the addition of any of the antagonists. Effectiveness of the Wnt inhibitors was observed by decreased levels of the established WNT-target gene AXIN2. Interestingly, the BMP antagonist Grem1 also down regulated AXIN2 albeit less strong than FRZb and DKK1 (Figure 5B). Addition of 20 ng/µ1 antagonists to differentiating hMSC's led to decreased levels of growth plate enriched genes such as Wnt5A, Wnt11, EPYC and PANX3 (Figure 5C).

## Claims

1. A method for evaluating the condition of a sample obtained from an individual, which sample comprises gene expression products of a cell residing in a joint of said individual, preferably a chondrocyte, said method comprising determining in said sample the expression levels of the genes Grem1, FRZb and DKK1.

2. Method according to claim 1, further comprising determining whether the said expression levels differ in comparison to the corresponding expression levels in a sample of a healthy control individual or in a sample of an unaffected joint (preferably the lateral joint) of the same individual.

3. Method according to any of the preceding claims, further comprising determining in said sample the gene expression level of at least one of the genes selected from the group of LTBP1, LTBP2, ID2, ID3, GDF10, Smad3, TGFβ3, Wnt5A, Wnt10B, Wnt11, FZD6, FZD8, FZD10, LEF1, GSK3B, Col3A1, Co15A2, KAL1, Col6A3, FBN1, Col14A1 Matn1, Col12A1, Co14A5, MFAP3, FBN3, Co14A6, CPXM1, CILP, DLK1, SRPX2, CHRDL2, LTBP2, CFH, MMP2, Wnt5A, WFDC1, genes coding for a Bone Morphogenic Protein (BMP) antagonist and genes coding for a Wnt antagonist.

4. Method according to any of the preceding claims, wherein said sample is a joint, a saliva, a blood, a serum, a urine or a synovial fluid sample.

5. Method according to any of the preceding claims, wherein said sample comprises a cell originating from articular cartilage, preferably a chondrocyte.

6. Method according to any of the preceding claims, wherein said individual is a mammal, preferably a human.

7. Method according to any of the preceding claims, further comprising a step of determining the levels of the uCTX-II and/or sCOMP proteins.

8. A method for determining whether an individual has an increased risk of
a. having a joint disease, or
b. developing symptoms of a joint disease or
c. developing symptoms associated with progression of a joint disease;
wherein said symptoms develop within 10 years after the completion of said method, wherein said method comprises performing the method of any one of claims 1-7 and further determining whether said condition is associated with an increased risk compared to the average risk of a reference population based on the comparison of the gene expression levels.

9. Method according to claim 8, wherein said joint disease comprises osteoarthritis, arthritis or rheumatoid arthritis (RA).

10. A kit of parts comprising up to 50 reagents, sequence specific oligonucleotides and/or capture agents for detecting gene expression products of Grem1, FRZb and DKK1.

11. Kit according to claim 10 further comprising at least one of the gene expression products of the genes listed in claim 3.

12. Method for determining whether a cartilage sample is a sample of articular cartilage or growth plate cartilage, said method comprising determining in said cartilage sample the expression level of the gene expression products of the genes Grem1, FRZb and DKK1.

13. Method for predicting the likelihood that a sample of cartilage tissue will undergo endochondral ossification when *implanted in vivo* by determining the levels of Grem1, FRZb and DKK1 gene expression products in said sample.

14. Grem1, FRZb and DKK1 proteins or an expression vector comprising a nucleic acid encoding Grem1, FRZb and DKK1 proteins for use in the treatment of a joint disease.
